Europäisches Patentamt

European Patent Office

Office européen des brevets

(11) Veröffentlichungsnummer: **0 491 234 A2**

## EUROPÄISCHE PATENTANMELDUNG

(21) Anmeldenummer: **91120807.2**

(51) Int. Cl.5: **G01N 19/02**, G01N 33/34

(22) Anmeldetag: **04.12.91**

(30) Priorität: **17.12.90 DE 4040250**

(43) Veröffentlichungstag der Anmeldung:
**24.06.92 Patentblatt 92/26**

(84) Benannte Vertragsstaaten:
**AT BE CH DE DK ES FR GB IT LI NL SE**

(71) Anmelder: **Stora Feldmühle
Aktiengesellschaft
Feldmühleplatz 1
W-4000 Düsseldorf 11(DE)**

(72) Erfinder: **Horand, Dieter, Dipl.-Phys.
Dorfstrasse 91
W-4055-Niederkrüchten(DE)**
Erfinder: **Wertschulte, Frank, Dipl.-Ing.
Freventstrasse 26a
W-4155 Grefrath 1(DE)**
Erfinder: **Altrogge, Getrud, techn. Ang.
Brentanostrasse 44
W-4052 Korschenbroich 2(DE)**

(54) **Messverfahren zur Bestimmung des Gleitreibungskoeffizienten von biegsamen, flächigen Bahnmaterialien und Messvorrichtungen zu seiner Durchführung.**

(57) Das Meßverfahren dient zur Bestimmung des Gleitreibungskoeffizienten von biegsamen, flächigen Bahnmaterialien, wie beispielsweise für Prüfstreifen A und B aus Papier. Die beiden zu messenden Prüfstreifen A und B werden mit ihren Enden parallel zur Zugrichtung befestigt und werden mit ihren losen Enden in entgegengesetzter Richtung durch einen quer zur Zugrichtung angeordneten, aus einem Walzenpaar (8;9) gebildeten Spalt geführt. Dabei werden die übereinanderliegenden Prüfstreifen A und B mit einer durch das Walzenpaar (8;9) und ggf. Auflagegewichten (11) vorbestimmten Auflagekraft $F_N$ belastet. Die eigentliche Messung erfolgt dadurch, daß die Prüfstreifen A und B parallel zur Zugrichtung mit konstanter Geschwindigkeit in entegengesetzer Richtung durch den Walzenspalt (14) bewegt werden, wobei mittels Kraftaufnehmer (5) die Reibkraft der aufeinandergleitenden Prüfstreifen A und B gemessen wird.

Fig. 5

EP 0 491 234 A2

Die Erfindung betrifft ein Meßverfahren zur Bestimmung des Gleitreibungskoeffizienten von biegsamen, flächigen Bahnmaterialien oder von einem biegsamen, flächigen Bahnmaterial und einem walzenförmigen Prüfkörper und eine Meßvorrichtung zu seiner Durchführung, bei denen die zu messenden gegeneinander reibenden Oberflächen unter einer vorbestimmten Andruckkraft im gleitenden Kontakt stehen.

Eine bekannte Meßanordnung zur Bestimmung des Gleitreibungskoeffizienten, insbesondere für Papierstreifen, besteht in der sogenannten Klötzchenmethode. Hierbei wird ein Papierstreifenabschnitt auf die plane Unterseite eines Klötzchens geklebt. Ein weiterer, langer Papierstreifen wird auf einer ebenen Unterlage verlegt und gegen Verschieben gesichert. Das mit dem Papierstreifenabschnitt präparierte Klötzchen bildet mit dem auf der Unterlage verlegten Papierstreifen, d. h. mit den zueinanderliegenden Papieroberflächen eine Reibpaarung. Das Klötzchen wird nun z.B. mittels geeigneter Vorschubeinrichtung über diese Unterlage mit konstanter Geschwindigkeit gezogen, wobei die am Klötzchen angreifende Zugkraftkomponente parallel zur Unterlage ausgerichtet sein muß. Die Zugkraft, welche gleichzeitig die Reibkraft ist, wird dabei mittels Kraftaufnehmer gemessen.

Der Gleitreibungskoeffizient läßt sich nach folgender Gleichung berechnen:

$\mu = F/F_N$

$F$ = Zugkraft (Reibkraft)

$F_N$ = Auflagekraft

$\mu$ = Gleitreibungskoeffizient

Zur genauen Bestimmung der Auflagekraft ($F_N$) muß das präparierte Klötzchen gewogen werden, um das Gewicht von Papierstreifenabschnitt und Kleber mit zu berücksichtigen.

Bei der Klötzchenmethode besteht der Nachteil, daß der auf dem Klötzchen aufgeklebte Papierstreifenabschnitt über die ganze Meßstrecke im reibenden Kontakt zur Unterlage steht und dabei durch Verschleiß seiner Oberfläche ständig einer veränderlichen Reibkraft unterliegt. Angaben zum Gleitreibungskoeffizienten von ähnlich gut gleitenden Papierbögen sind nur bedingt möglich, denn es lassen sich oft keine signifikanten Unterschiede bei den Meßergebnissen feststellen. Weitere Nachteile der Klötzchenmethode ergeben sich aus der vorgegebenen Klötzchenunterseite, die mit einem Papierstreifen zu präparieren ist, so daß die Messung nur den mittleren Gleitreibungskoeffizienten der ganzen, durch die Klötzchenunterseite festgelegten, Prüffläche erfassen kann. Deshalb können partiell unterschiedliche Reibwerte einer solchen Prüffläche nicht gemessen werden, wie sie beispielsweise durch ein auf das Papier aufgedrucktes Druckbild zu erwarten wären. Auch ergibt sich ein Unsicherheitsfaktor im Meßergebnis daraus, daß das Verwenden von Kleber zum Befestigen der Prüfstreifen Einfluß auf sein Material und damit auf sein Gleitverhalten haben kann.

Aus dem Stand der Technik sind nachfolgend Ausführungsformen von Meßvorrichtungen bekannt, die aber prinzipiell auf die Klötzchenmethode zurückgeführt werden können und deshalb die vorgenannten Nachteile nicht überwinden können.

Die aus der DDR-Patentschrift 126 542 bekannte Einrichtung zur Messung der Reibungskräfte von bahnförmigen Materialien betrifft eine Bandführung mit einer auf federnden Stützen beweglichen Auflagefläche, über die das bahnförmige Material transportiert wird. Die Reibkraft zwischen Auflagefläche und relativ dazu bewegtem bahnförmigem Material wird gemessen. Der Unterschied zur Klötzchenmethode besteht darin, daß das Band bewegt wird und nicht die Auflagefläche, welche gedanklich das Klötzchen darstellt.

Die aus GB-2205165 A1 und US-PS 4000641 bekannten Vorrichtungen zur Reibungsmessung von über zylindrischen Oberflächen gleitendem Garn geben die Differenz der Garnspannung vor und nach Kontakt mit der Prüfoberfläche als Maß für die Reibung an. Nachteilig ist, abgesehen von der während des Reibvorganges Überstreichen des Garns gleicher Prüfoberflächenbereiche, die sich durch die Zugkraft des Garns selbständig einstellende Normalkraft. Daher würde sich mit einer solchen Vorrichtung bei der Reibungsmessung von bahnförmigem Material eine zu geringe Normalkraftkomponente einstellen.

Ein aus der CH-661 594 A5 bekanntes Verfahren, sowie bekannte Vorrichtung zum Feststellen des Reibungsverhaltens von langgestreckten Gebilden, insbesondere von Garnen betrifff die in Abhängigkeit der Abzugsgeschwindigkeit des Garns Ermittlung der Garndehnung und der auf das Garn einwirkenden Zugkraft. Der Reibungskoeffizient des Garns ist meist unbekannt, da es sich bei der Reibung von Garnen gegen einen Reibkörper um eine Art Mischreibung zwischen Festkörperreibung und Flüssigkeitsreibung handelt. Der Nachteil dieser bekannten Vorrichtung liegt im Bremskopf begründet, der weniger geeignet ist, bahnförmige Materialien, wie z.B. Papierstreifen, aufzunehmen. Der Bremskopf bewirkt immer eine gleichzeitige Reibwirkung auf die Ober- und Unterseite des durchgezogenen Materials, zu dem staut sich anfallender Abrieb des Materials im Einlaß des Bremskopfes und wirkt sich auf das Meßergebnis aus.

Die Erfindung sieht ihre Aufgabe darin, die bestehenden, insbesondere durch das Aufbringen eines flächigen Prüfstreifenabschnittes auf die Klötzchenunterseite mit der sich während der Messung verändernden Kontaktfläche entstehenden, Nachteile zu überwinden. Dabei sollen Meßverfahren und Meßvorrichtun-

2

gen zur Durchführung der Meßverfahren zur Verfügung gestellt werden, mit deren Hilfe auf einfache Weise, eine genauere als die bisher mit der Klötzchenmethode mögliche Bestimmung des Gleitreibungskoeffizienten ermöglicht wird und der Gleitreibungskoeffizient einerseits auf lokal eng begrenzte Prüfflächen und andererseits über lange Prüfflächen bestimmbar ist und ferner soll ein Meßverfahren zur Verfügung gestellt werden, das unter zur Hilfenahme einer Meßvorrichtung die Bestimmung des Gleitreibungskoeffizienten von einem mit einem Prüfkörper im reibenden Kontakt stehenden Prüfstreifen ermöglicht.

Zur Lösung der Aufgabe sieht die Erfindung bei einem Meßverfahren gemäß Patentanspruch 1 vor, daß zum Bestimmen des Gleitreibungskoeffizienten von biegsamen, flächigen Bahnmaterialien, insbesondere von Druckpapieren, sowie Verpackungsfolien aus Kunststoff oder Metall, die Reibkraft der aufeinanderliegenden im gleitenden Kontakt stehenden Bahnen gemessen wird. Dabei stehen die gegeneinander reibenden Oberflächen der Bahnmaterialien unter einer vorbestimmten Andruckkraft, d.h. die Auflagekraft ist bekannt und wird während des Meßvorgangs nicht verändert. Die Durchführung des Meßverfahrens erfolgt dadurch, daß zwei Prüfstreifen des Bahnmaterials mit einem ihrer Enden parallel zur Zugrichtung befestigt werden und mit ihren losen Enden in entgegengesetzter Richtung durch einen quer zur Zugrichtung angeordneten, aus einem Walzenpaar gebildeten Spalt geführt werden, daß die beiden im Walzenspalt übereinanderliegenden Prüfstreifen mit einer durch das Walzenpaar vorbestimmten Auflagekraft belastet werden und daß die Prüfstreifen parallel zur Zugrichtung mit konstanter Geschwindigkeit bewegt werden, wobei mittels Kraftaufnehmer die Zugkraft gemessen wird, während die aufeinanderliegenden Prüfstreifen durch den Walzenspalt in entgegengesetzter Richtung gleiten.

Die beim erfindungsgemäßen Meßverfahren verwendeten Prüfstreifen bleiben, so wie sie als Probe entnommen wurden, unverändert, d.h. es braucht z.B. kein Kleber aufgetragen zu werden. Es ist zweckmäßig, zunächst beide Prüfstreifen für sich alleine zu messen. Dabei wird jeweils der zu messende Prüfstreifen genauso eingelegt, als ob beide Prüfstreifen zusammen gemessen würden. Auch die mittels Walzenpaar aufgebrachte Druckkraft im Walzenspalt entspricht derjenigen, als ob beide Prüfstreifen übereinanderliegend durch den Spalt gezogen würden. Die aus den beiden unter der Bezeichnung Null-Versuch gemessenen Zugkräfte berücksichtigen die bei der späteren Berechnung des Gleitreibungskoeffizienten durch die Meßvorrichtung bedingten Widerstandskräfte. Diese Widerstandskräfte resultieren aus Reibkräften von Führungsteilen und aus der Walkarbeit im Walzenspalt.

Nach Durchführung der Null-Versuche wird im Hauptversuch die Zugkraft mit beiden Prüfstreifen gemessen. Vorzugsweise werden die mittels Kraftaufnehmer gemessenen Zugkräfte aus dem Hauptversuch mit denen aus den Null-Versuchen gemeinsam auf einem Meßschrieb zur späteren Auswertung aufgezeichnet.

Die Meßstrecke, d. h. die Länge der Prüfstreifen, kann je nach Versuchsanforderung festgelegt werden, sofern die Zugeinrichtung dafür geeignet ist. In der Praxis haben sich Prüfstreifenabmessungen bis 50 mm Breite und ca. 150 mm Länge bewährt.

Ein besonderer Vorteil des erfindungsgemäßen Meßverfahrens ist in der gegenläufigen Anordnung der Prüfstreifen zu sehen, denn solange sich die Prüfstreifen parallel zur Zugrichtung bewegen und aufeinander gleitend in entgegengesetzter Richtung durch den Spalt gezogen werden, reiben fortlaufend die bis dahin noch nicht im Kontakt stehenden Prüfstreifenoberflächen miteinander. Es tritt daher kein nennenswerter Verschleiß der Prüfstreifenoberflächen auf, der auf das Meßergebnis einen Einfluß haben könnte. Zur Bestimmung eines statistisch gesicherten Ergebnisses werden vorzugsweise die Gleitreibungskoeffizienten aus 10 Vergleichsmessungen herangezogen, um daraus die Standardabweichung des Gleitreibungskoeffizienten zu errechnen.

Ein weiterer Vorteil der Erfindung ergibt sich aufgrund der kleinen Kontaktfläche im Walzenspalt. Es können Reibkräfte in lokal eng begrenzten Bereichen gemessen werden, wie sie im Beispiel 2 anhand eines mit Einzelfarben bedruckten Prüfstreifens aus Papier gezeigt werden.

Zur Bestimmung des Gleitreibungskoeffizienten von einem biegsamen, flächigen Bahnmaterial und einem walzenförmigen Prüfkörper, bei dem die zu messenden gegeneinander reibenden Oberflächen unter einer vorbestimmten Andruckkraft im gleitenden Kontakt stehen, sieht die Erfindung bei einem Meßverfahren gemäß Patentanspruch 2 folgende Verfahrensschritte vor:

a) ein Prüfstreifen des Bahnmaterials wird mit einem seiner Enden parallel zur Zugrichtung befestigt und mit seinem losen Ende durch einen quer zur Zugrichtung angeordneten, aus einem Walzenpaar gebildeten Walzenspalt geführt, wobei eine der Walzen zur Aufnahme des walzenförmigen Prüfkörpers dient;

b) der im Walzenspalt liegende Prüfstreifen wird mit einer durch das Walzenpaar vorbestimmten Auflagekraft belastet;

c) gleichzeitig wird der Prüfstreifen parallel zur Zugrichtung mit konstanter Geschwindigkeit bewegt, wobei die mit dem Prüfkörper versehene Walze mittels drehfester Lagerung gehalten oder entgegen der

EP 0 491 234 A2

Bewegungsrichtung des Prüfstreifens mit konstanter Winkelgeschwindigkeit gedreht wird, so daß zwischen Prüfstreifenoberfläche und Prüfkörperoberfläche eine Relativbewegung stattfindet;

d) mittels Kraftaufnehmer wird die Zugkraft gemessen, während sich der Prüfstreifen durch den Walzenspalt bewegt und auf der mit dem walzenförmigen Prüfkörper versehenen Walze gleitet.

Vorzugsweise wird die mittels Kraftaufnehmer gemessene, parallel zur Prüfstreifenbefestigung ausgerichtete Zugkraftkomponente auf einem Meßschrieb zur späteren Auswertung aufgezeichnet.

Auch hier ist es zweckmäßig zunächst die durch die Meßanordnung bedingten Widerstandskräfte wieder im Null-Versuch zu messen und aufzuzeichnen, dabei muß die mit dem Prüfkörper bestückte Walze in Bewegungsrichtung des Prüfstreifens bei möglichst geringer Lagerreibung mitdrehen können.

Im Hauptversuch erfolgt dann die Messung der Zugkraft bzw. Reibkraft vorzugsweise mit drehfest gelagertem Prüfkörper, wenn dadurch eine in der Praxis vergleichbare Situation dargestellt werden kann, wie es z.B. bei über das Papier gleitende Keramikschreibköpfe von Computerdruckern der Fall ist. Soll dagegen das Zusetzen einer rauhen Prüfkörperoberfläche mit Bahnmaterialrückständen und sich dadurch veränderndes Reibverhalten verhindert werden, sieht das erfindungsgemäße Meßverfahren vor, daß die Walze mit aufgestecktem Prüfkörper entgegen der Bewegungsrichtung des Prüfstreifens gedreht wird, vorzugsweise mit der gleichen Oberflächengeschwindigkeit wie die des Prüfstreifens, so daß auch hier fortlaufend die bis dahin noch nicht im Kontakt stehenden Oberflächen von Prüfstreifen und Prüfkörper miteinander reiben.

Eine Meßvorrichtung mit vertikaler Zugvorrichtung zur Durchführung der Meßverfahren sieht die Erfindung gemäß Patentanspruch 3 vor, die im wesentlichen aus einer mit einem Verbindungteil versehenen Halterung, die in Zugrichtung der vertikal verfahrbaren Zugvorrichtung aufhängbar ist, einem zwischen Verbindungsteil und Zugvorrichtung angeordneten Kraftaufnehmer, einem Ständer zur Aufnahme der Zugvorrichtung sowie zwei entgegen der Zugrichtung auf dem Ständer angebrachten Befestigungsmittel zum Einspannen von Prüfstreifen A und B besteht. Auf der Halterung sind eine Zentralwalze und eine von der Zentralwalze abhebbare Auflagewalze gelagert, die zusammen einen horizontalen, zur Zugrichtung senkrecht angeordneten Walzenspalt bilden. Eine auf der Halterung gelagerte Umlenkwalze, die zum Umführen des oberen Prüfstreifens B aus der Horizontalen in die Vertikale dient.

Den Kern der erfindungsgemäßen Meßvorrichtung bildet die Halterung mit der auf ihr gelagerten Zentralwalze und mit der vorzugsweise in einem Hebel gelagerten und damit von der Zentralwalze abhebbaren Auflagewalze. Der Hebel ist mit seinem Drehpunkt ebenfalls auf der Halterung gelagert. Zusammen bilden Zentralwalze und Auflagewalze einen horizontalen Walzenspalt, der senkrecht zur Zugrichtung angeordnet ist. Gegenüber vom Hebel, in Höhe des Walzenspaltes, ist auf der Halterung die Umlenkwalze gelagert.

Die Halterung wird vorzugsweise in einer bekannten Zugvorrichtung, die zum vertikalen Verfahren der Halterung dient, eingehängt, wie sie z.B. für Zugversuche an Papierproben mit einer Zugprüfmaschine nach DIN 51221 zu verwenden ist. Ein Verbindungsteil, das an der Halterung in Zugrichtung oberhalb vom Walzenspalt angeordnet ist, bildet die Aufhängung bzw. die Schnittstelle zur Zugvorrichtung. Die Zugprüfmaschine nach DIN 51221 besitzt einen kräftigen Ständer mit Ständerfuß und weist einen Kraftaufnehmer auf, der zwischen Verbinungsteil und Zugvorrichtung angeordnet ist. Die Befestigungsmittel für die Prüfstreifen A und B sind entgegen der Zugrichtung auf dem Ständerfuß angebracht und müssen so ausgebildet sein, daß beim Einspannen der Prüfstreifen keine Vorspannung eintritt und die Prüfstreifen beim Belasten nicht rutschen.

Der untere Prüfstreifen A wird vom Befestigungsmittel aus senkrecht nach oben geführt, umschlingt dann die Zentralwalze mit etwa 90 Grad, so daß er aus dem Walzenspalt tangential hervortritt und aufgrund der Schwerkraft bogenförmig nach unten durchhängt. Der obere Prüfstreifen B wird vom anderen Befestigungsmittel aus senkrecht nach oben geführt und mittels Umlenkwalze in die Waagerechte umführt, so daß er schließlich entgegengesetzt zu Prüfstreifen A auf der anderen Seite des Walzenspaltes tangential hervortritt. Dadurch, daß der obere Prüfstreifen B von der Umlenkwalze aus waagerecht, ohne Prüfstreifen A zu berühren, in den Walzenspalt geführt wird, ist sichergestellt, daß die unter Last zu messenden gegeneinander wirkenden Prüfstreifenoberflächen nur im definierten Walzenspalt Kontakt haben.

Der Gleitreibungskoeffizient errechnet sich aus folgender Gleichung bei Vernachlässigung aller durch die Meßvorrichtung bedingten Widerstandskräfte:

$\mu_G = F / 4F_N$

F = Zugkraft

$F_N$ = Auflagekraft

Bei Berücksichtigung der Widerstandskräfte:

$\mu_G = (F - F_o)/(4F_N)$

F = Zugkraft aus Hauptversuch

4

EP 0 491 234 A2

$F_o$ = Widerstandskraft addiert aus beiden Null-Versuchen

$F_N$ = Auflagekraft

$\mu_G$ = Gleitreibungskoeffizient

Eine alternative Meßvorrichtung mit horizontaler Zugvorrichtung zur Durchführung der Meßverfahren sieht die Erfindung gemäß Patentanspruch 7 vor, die im wesentlichen aus einem auf einem Führungsbett mittig angeordneten Walzenständer, zwei auf dem Führungsbett vom Walzenständer aus nach außen linear verfahrbarer Schlitten mit Befestigungsmittel für Prüfstreifen, sowie einem auf einen der beiden Schlitten angeordneten Kraftaufnehmer besteht.

Auf dem Walzenständer sind die Zentralwalze und die von ihr abhebbare Auflagewalze gelagert, die zusammen einen quer zur Zugrichtung angeordneten Walzenspalt bilden. Am Walzenständer sind Mittel zum Führen der Prüfstreifen angebracht, damit sichergestellt wird, daß sich die Prüfstreifen nur im Walzenspalt berühren.

Die linear verfahrbaren Schlitten werden vorzugsweise mit einem Spindelantrieb verbunden, der die Schlitten gleichzeitig mit konstanter Geschwindigkeit nach außen verfahren kann. Damit die Reibkraft der aufeinandergleitenden Prüfstreifen parallel zur Zugrichtung gemessen werden kann, sind die Befestigungsmittel so auf den Schlitten angebracht, daß sie mit dem Walzenspalt fluchten.

Der Gleitreibungskoeffizient $\mu_G$ berechnet sich direkt aus der Zugkraft (Reibkraft) F und der auf ihr senkrecht wirkenden Auflagekraft $F_N$ mit der Gleichung:

$\mu_G$ = $F/F_N$

Bei Berücksichtigung der Widerstandskräfte mit $F_O$:

$\mu_G$ = $(F - F_O)/F_N$

Eine besonders vorteilhafte Ausführungsform der Erfindung sieht vor, daß die Auflagewalze und/oder die Zentralwalze gummierte Walzenmäntel aufweisen. Gummierte Walzenmäntel nehmen aufgrund ihrer Elastizität kleine Unebenheiten der Prüfstreifen auf und dämpfen so Schwankungen der gemessenen Zugkraft ab, so daß im Hauptversuch die Reibkräfte der gegeneinander gleitenden Prüfstreifen weniger stark überlagert erscheinen. Auch werden durch elastische Walzenmäntel geringere spezifische Druckkräfte im Walzenspalt bei gleichem Auflagegewicht der Auflagewalze erzeugt als dies bei harten Walzen der Fall wäre. Prüfstreifen aus empfindlichen Materialien werden so geringer beansprucht und es erfolgen keine Veränderungen der Prüfstreifenmaterialien, welche sich auf den Gleitreibungskoeffizienten auswirken könnten.

Bei einer weiteren Ausführung der erfindungsgemäßen Meßvorrichtung weist die Zentralwalze einen walzenförmigen Prüfkörper auf, der beim Meßverfahren gemäß Patentanspruch 2 mit einem Prüfstreifen im reibenden Eingriff steht. Das Befestigen des Prüfkörpers mittels Paßfeder hat den Vorteil, daß auf einfache Weise Prüfkörper ausgetauscht und gegen Verdrehen gesichert werden können. Zum Messen des Reibwertes zwischen Prüfkörperoberfläche und Prüfstreifenoberfläche muß eine Relaivbewegung stattfinden. Die Zentralwalze wird mittels Kernantrieb mit konstanter Winkelgeschwindigkeit gedreht, wobei die Tangentialgeschwindigkeit der Prüfkörperoberfläche entgegen der Bewegungsrichtung des Prüfstreifens gerichtet ist oder durch Festhalten der Zentralwalze die Tangentialgeschwindigkeit der Prüfkörperoberfläche gleich Null ist.

Die nachfolgenden Beispiele zeigen die Leistungsfähigkeit des erfindungsgemäßen Meßverfahrens:

Beipiel 1 zeigt eine Gegenüberstellung der Gleitreibungskoeffizienten, die zum einen mit der Klötzchenmethode, und zum anderen mit dem erfindungsgemäßen Meßverfahren ermittelt wurden.

Beispiel 2 zeigt lokale Meßergebnisse von Prüfstreifen aus Papier mit Teilflächendruck.

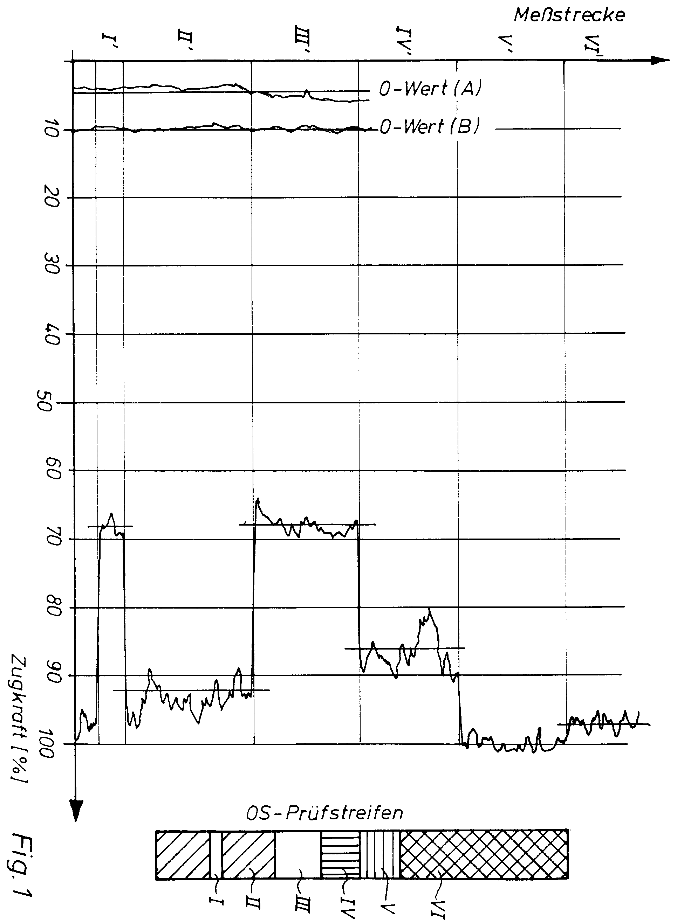

Fig. 1 zeigt einen über die Meßstrecke aufgezeichneten Meßschrieb zum Beispiel 2.

Im Beispiel 1 wurden Prüfstreifen aus dem gleichen Papier untersucht, wobei die Reibkraft zwischen unbedruckter Oberseite (OS) und Siebseite (SS) gemessen wurde. Durchgeführt wurden 3 Versuchsreihen mit jeweils 10 Einzelmessungen:

Versuch 1: mit der Klötzchenmethode,

Versuch 2: mit der Walzenmethode, d.h. mit dem erfindungsgemäßen Meßverfahren, ohne zusätzliches Auflagegewicht ($F_N$ = 5,3 N),

Versuch 3: mit der Walzenmethode mit 200 g zusätzliches Auflagegewicht ($F_N$ = 7,3 N).

Prüfmaschinengeschwindigkeit: 100mm/min

Prüfstreifenabmessungen für Versuche 2 und 3:

Prüfstreifen A: 50 mm breit und 130 mm lang,

Prüfstreifen B: 40 mm breit und 130 mm lang.

Die unterschiedlichen Prüfstreifenbreiten verhindern beim erfindungsgemäßen Meßverfahren Meßfehler aufgrund sich verändernder Kontaktflächenbreiten im Walzenspalt durch ggf. geringfügiges Schräglaufen der Prüfstreifen.

5

Die aus den Meßergebnissen errechneten Gleitreibungskoeffizienten sind in nachfolgender Tabelle 1 aufgeführt.

## Tabelle 1

Gleitreibungskoeffizienten für Papier OS/SS

| Versuch 1: | | Versuch 2: | | Versuch 3: | |
|---|---|---|---|---|---|
| Klötzchenmethode | | Walzenmethode ohne zusätzliches Auflagegewicht | | Walzenmethode mit 200 g Auflagegewicht | |
| 1.Wert | 0.366 | 1.Wert | 0.206 | 1.Wert | 0.210 |
| 2.Wert | 0.361 | 2.Wert | 0.211 | 2.Wert | 0.212 |
| 3.Wert | 0.340 | 3.Wert | 0.215 | 3.Wert | 0.223 |
| 4.Wert | 0.314 | 4.Wert | 0.219 | 4.Wert | 0.210 |
| 5.Wert | 0.330 | 5.Wert | 0.215 | 5.Wert | 0.211 |
| 6.Wert | 0.356 | 6.Wert | 0.217 | 6.Wert | 0.211 |
| 7.Wert | 0.366 | 7.Wert | 0.213 | 7.Wert | 0.220 |
| 8.Wert | 0.314 | 8.Wert | 0.215 | 8.Wert | 0.217 |
| 9.Wert | 0.366 | 9.Wert | 0.215 | 9.Wert | 0.218 |
| 10.Wert | 0.340 | 10.Wert | 0.217 | 10.Wert | 0.212 |

Mittelwert:

| 0.345 | 0.214 | 0.214 |
|---|---|---|

Standardabweichung:

| 0.02 | 0.004 | 0.003 |
|---|---|---|

Variationskoeffizient %:

| 5.8 | 1.9 | 1.4 |
|---|---|---|

Tabelle 1 zeigt, daß der Variationskoeffizient (prozentuale Standardabweichung vom Mittelwert) bei den Versuchen 2 und 3 deutlich niedriger als bei dem Versuch 1 ist.

Durch die Erhöhung des Auflagegewichtes ändert sich beim erfindungsgemäßen Meßverfahren der Gleitreibungskoeffizient nicht, wie aus Versuch 3 zu entnehmen ist.

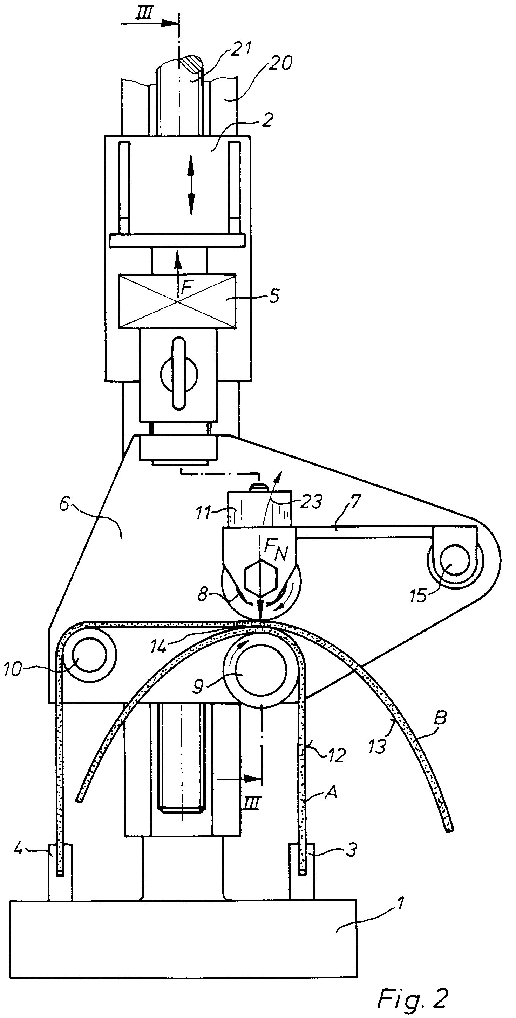

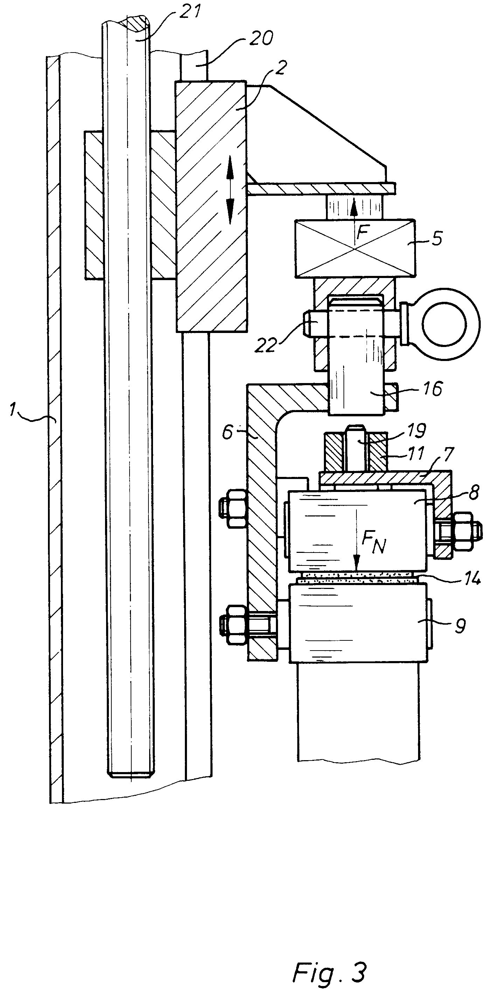

Die Reibkräfte zu den Versuchen 2 und 3 wurden mit einer Meßvorrichtung mit vertikaler Zugvorrichtung ermittelt, wie sie nachfolgend anhand der Figuren 2 und 3 gezeigt wird. Diese erfindungsgemäße Meßvorrichtung, geeignet zum Einbau in einer Zugprüfmaschine nach DIN 51221, ermöglicht eine genauere reproduzierbare Messung der Reibkräfte als es bisher mit der Klötzchenmethode möglich war. Auch zeigen die Ergebnisse, daß der Gleitreibungskoeffizient nicht von der Auflagekraft, sondern nur, wie es die Theorie vorgibt, von der Materialpaarung abhängig sein darf.

Im Beispiel 2 wurden zwei Prüfstreifen aus Papier untersucht. Die Prüfstreifen von 20mm Breite und 150mm Länge weisen folgenden Teilflächendruck auf (OS = Oberseite; SS = Siebseite):

1. Prüfstreifen OS:     unbedruckt - bedruckt beige - unbedruckt - bedruckt blau - bedruckt grün - bedruckt schwarz.

2. Prüfstreifen SS:    unbedruckt.

Die Reibpaarung zwischen bedruckter OS und unbedruckter SS ist in der Praxis häufig anzutreffen. Die Ergebnisse sind in Tabell 2 aufgeführt und Fig. 1 zeigt den dazugehörigen Meßschrieb.

Tabelle 2

| Gleitreibungskoeffizienten für Papier mit Teilflächendruck OS/SS | | |
|---|---|---|
| Teilbereich | Motivstelle | μG |
| I | unbedruckt | 0.25 |
| II | bedruckt beige | 0.37 |
| III | unbedruckt | 0.25 |
| IV | bedruckt blau | 0.34 |
| V | bedruckt grün | 0.40 |
| VI | bedruckt schwarz | 0.39 |

Aus Tabelle 2 geht hervor, daß sich der Gleitreibungskoeffizient zwischen bedruckter OS und unbedruckter SS gegenüber unbedruckter OS und unbedruckter SS stark erhöht, wobei die Druckfarben unterschiedliche Gleitreibungskoeffizienten aufweisen. Informationen dieser Art auf lokal eng begrenzten Prüfflächen sind mit der Klötzchenmethode nicht zu erhalten.

Fig. 1 zeigt den Meßschrieb auf dem der mit Motivstellen bedruckte Prüfstreifen mit seiner Oberseite OS, wie im Beispiel 2 beschrieben, schematisch eingezeichnet ist. Die Teilbereiche der Motivstellen sind mit römischen Zahlen gekennzeichnet (I bis VI).

Der Meßschrieb zeigt auf seiner Abszissenachse die Zugkraft in %, wobei 100% = 10 N entsprechen und auf seiner Ordinatenachse die Meßstrecke. Das Verhältnis zwischen Motivlänge und Meßschrieblänge entspricht dem der Teilbereiche (I bis VI und I^ bis VI^).

Die gemessenen Zugkräfte, links die beiden O-Werte aus den Null-Versuchen, sowie rechts die wesentlich größeren Werte aus dem Hauptversuch, wurden bei einer Auflagekraft von 5,3 N ermittelt.

Der kleinere O-Wert resultiert aus dem Null-Versuch mit Prüfstreifens A, wobei Prüfstreifen A nur durch den Walzenspalt gezogen wurde. Der obere Prüfstreifen B wurde über die Umlenkwalze und durch den Walzenspalt gezogen.

Zur Auswertung der Meßergebnisse wurden die Zugkräfte jedes Teilbereichs gemittelt, siehe Geraden parallel zur Ordinatenachse. Die rechnerisch ermittelten Gleitreibungskoeffizienten sind aus der Tabelle 2 zu entnehmen.

Ausführungsformen der Meßvorrichtung wird anhand der Zeichnung beschrieben. Es zeigen

Fig. 2    die Vorderansicht einer Meßvorrichtung mit vertikaler Zugvorrichtung zur Durchführung des erfindungsgemäßen Meßverfahrens,

Fig. 3    einen Schnitt entlang der Linie III/III durch die in Fig. 2 gezeigte Meßvorrichtung,

Fig. 4    einen Ausschnitt der in Fig.2 gezeigten Meßvorrichtung alternativ mit Zentralwalzenantrieb.

Fig. 5    die Vorderansicht einer alternativen Meßvorrichtung mit horizontaler Zugvorrichtung zur Durchführung des erfindungsgemäßen Meßverfahrens.

Die in Figur 2 und 3 gezeigte Meßvorrichtung weist einen stabilen Ständer (1) auf, der eine über den Ständerfuß beginnende senkrecht nach oben gerichtete Flachführung (20) zur Aufnahme der Zugvorrichtung (2) besitzt. Die Zugvorrichtung (2) hängt in einem nicht vollständig dargestellten Spindeltrieb (21), der zum schnellen und langsamen Verfahren der Zugvorrichtung (2) in vertikaler Richtung dient.

Auf dem Ständerfuß befinden sich zwei Befestigungsmittel (3; 4), die das eine Ende von Prüfstreifen A und B über deren gesamte Breite festhalten, wobei in Fig. 2 die Dicke der Prüfstreifen stark übertrieben dargestellt ist. Befestigungsmittel (3) ist so ausgerichtet, daß Prüfstreifen A senkrecht nach oben gerichtet dabei Zentralwalze (9) tangential berührt und diese etwa mit 90 Grad umschlingt, so daß sein loses Ende auf der linken Seite, d.h. durch den Walzenspalt (14) hindurch, heraustritt. Prüfstreifen B ist ausgehend vom Befestigungsmittel (4) zunächst senkrecht nach oben gerichtet, dann über Umlenkwalze (10) in die Waagerechte geführt, so daß Prüfstreifen B über A liegend von links nach rechts, also entgegengesetzt zu A, aus dem Walzenspalt (14) heraustritt. Die Prüfstreifen A und B berühren sich unter Last ($F_N$) mit ihren Oberflächen (12; 13) nur unmittelbar im Walzenspalt (14).

Umlenkwalze (10) und Zentralwalze (9) sind auf der Halterung (6) drehbar gelagert. Ihre Walzenmäntel sind gummiert und parallel zueinander ausgerichtet. Die ebenfalls gummierte Auflagewalze (8), die mit der Zentralwalze (9) den Walzenspalt (14) bildet, ist im Hebel (7) drehbar gelagert. Einstellmittel, die zum parallelen Ausrichten des Walzenspaltes (14) dienen, sind nicht näher dargestellt, befinden sich aber zweckmäßigerweise im Drehpunkt (15) der Hebellagerung. Der Hebel (7) ist mit seinem Drehpunkt (15) auf der Halterung (6) angeordnet und in Pfeilrichtung (23) schwenkbar. Die Auflagekraft $F_N$ ergibt sich aus der Gewichtskraft von Auflagewalze (8) und Hebel (7), sowie Zusatzgewicht (11), wobei ihre Wirklinie senkrecht zum horizontalen Walzenspalt (14) gerichtet ist. Zentrierdorn (19) plaziert das Zusatzgewicht (11) auf dem Hebel (7), so daß dessen Gewichtskraft genau in Richtung zur Wirklinie $F_N$ wirkt.

In der Halterung (6) ist ein zylindrisches Verbindungsteil (16) angebracht. Das über die Halterung (6) hinausragende Verbindungsteil (16) ist in einer Aufnahmebohrung der Zugvorrichtung (2) eingelassen und Bolzen (22) sichert das Ganze gegen Herausfallen. Die so eingehängte Halterung (6) kann durch Herausziehen des Bolzens (22) auf einfache Weise ausgebaut werden und ggfs. an einem anderen Ort in einer ähnlichen Zugvorrichtung eingebaut werden. Der Kraftaufnehmer (5) zur Messung der Zugkraft F ist Bestandteil der Zugvorrichtung (2).

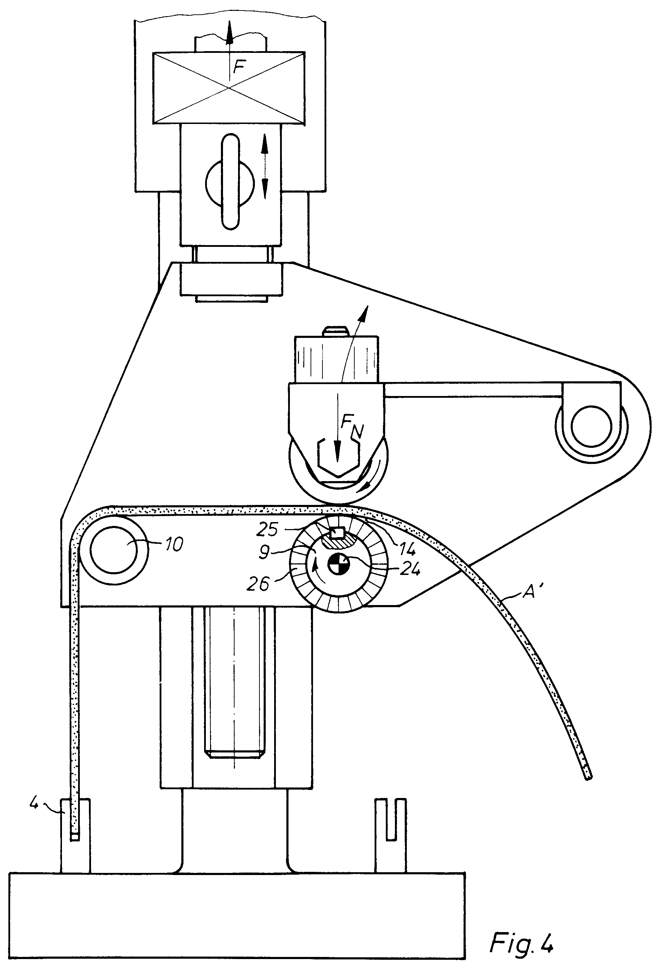

Bei der in Fig. 4 gezeigten Ausführung dient ein auf der Zentralwalze (9) aufgeschobener, walzenförmiger Prüfkörper (26) als Reibpartner zu Prüfstreifen A'. Die Zentralwalze (9) verfügt über einen sich im Uhrzeigersinn drehenden Zentrumsantrieb (24). Die Paßfeder (25) ergibt eine billige und einfach zusammen zu bauende und leicht wieder zu lösende Verbindung mit dem Prüfkörper (26) und überträgt das vom Zentrumsantrieb (24) aufgebrachte Drehmoment formschlüssig. Während des Meßvorgangs gleitet der Prüfstreifen durch den Walzenspalt (14) und zwar entgegen der Drehrichtung von Zentralwalze (9), so daß zwischen den gegeneinander reibenden Oberflächen von Prüfstreifen A' und Prüfkörper (26) eine Relativbewegung stattfindet. Prüfstreifen A' ist mit Befestigungsmittel (4) verbunden, mittels Umlenkwalze (10) aus der Horinzontalen in die Vertikale geführt und tritt schließlich tangential aus dem Walzenspalt (14) hervor, wobei die zu messenden gegeneinander wirkenden Oberflächen nur im definierten Walzenspalt durch die Auflagekraft $F_N$ belastet werden.

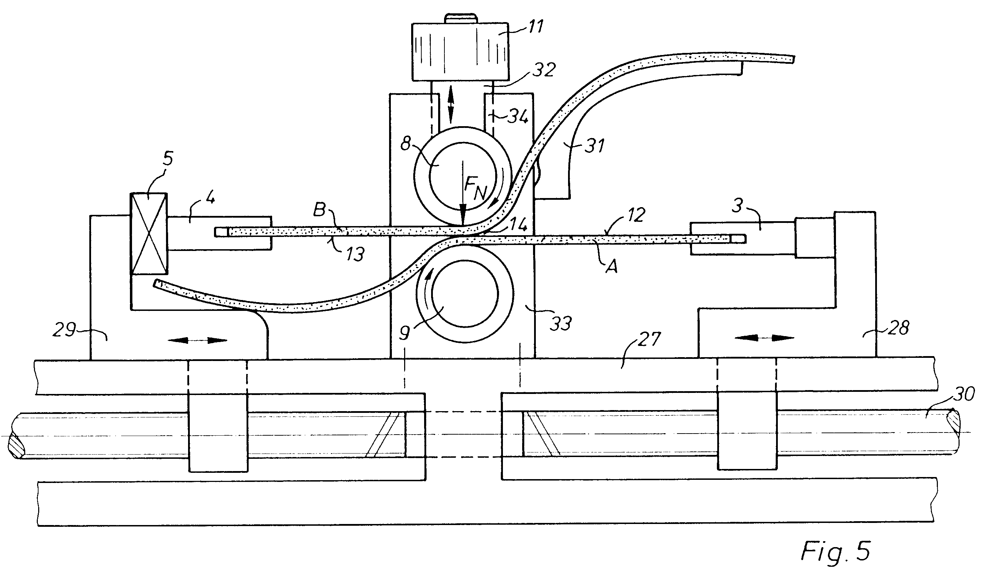

Fig. 5 zeigt eine alternative Ausführung der Meßvorrichtung mit horizontaler Zugvorrichtung. Hier wird nicht das Walzenpaar bestehend aus Zentralwalze (9) und Auflagewalze (8) bewegt, sondern die zur Aufnahme der Prüfstreifen A und B dienenden Befestigungsmittel (3 und 4).

Die Zugvorrichtung besteht im wesentlichen aus einem langen Führungsbett (27), das in seiner Mitte den Walzenständer (33) trägt, sowie zwei durch eine Bewegungsspindel (30) mit Rechts- und Linksgewinde entgegengesetzt angetriebene Schlitten (28; 29), die auf der Oberseite des Führungsbettes (27) gleiten und sich gemeinsam vom Walzenständer aus nach außen oder wieder zur Mitte hin verfahren lassen. Die Befestigungsmittel (3; 4) fluchten genau mit dem Walzenspalt (14), so daß die Prüfstreifen A und B parallel zur Zugkraftkomponente gezogen werden können. Zwischen Befestigungsmittel (4) und Schlitten (29) ist der zur Messung der Zugkraft F vorgesehene Kraftaufnehmer (5) angeordnet. Da die Zugkraft F für beide Prüfstreifen gleichgroß ist, könnte der Krafaufnehmer (5) auch auf der anderen Seite, d.h. zwischen Befestigungsmittel (3) und Schlitten (28) angebracht werden. Bei Bestimmung der durch die Meßvorrichtung bedingten Widerstandskräfte $F_o$ reicht es hier aus, nur den oberen Prüfstreifen B im Null-Versuch zu messen, womit die bevorzugte, wie in Figur 5 gezeigte, Anordnung des Kraftaufnehmers (5) festliegt.

Im Walzenständer (33) ist die Zentralwalze (9) drehbar gelagert und bildet mit der Auflagewalze (8) den Walzenspalt (14), wobei die auf dem Führungsteil (32) ebenfalls drehbar gelagerte Auflagewalze (8) im Walzenständer in der Führung (34) geführt wird und durch Verschieben nach oben von der Zentralwalze (9) abgehoben werden kann. Die Auflagekraft $F_N$ ergibt sich aus dem Eigengewicht von Auflagewalze (8) einschl. Führungsteil (32) und dem Zusatzgewicht (11), wobei ihre Wirklinie senkrecht zur Zentralwalze (9) verläuft.

Das am Walzenständer (33) auf der rechten Seite oberhalb vom Walzenspalt (14) angeordnete Leitblech (31) trägt den oberen Prüfstreifen B, so daß Prüfstreifen A und B sich während der Messung mit ihren gegeneinander reibenden Oberflächen (12 und 13) nur unmittelbar im Walzenspalt (14) berühren.

**Patentansprüche**

1. Meßverfahren zur Bestimmung des Gleitreibungskoeffizienten von biegsamen, flächigen Bahnmaterialien, bei denen die zu messenden gegeneinander reibenden Oberflächen unter einer vorbestimmten Andruckkraft im gleitenden Kontakt stehen, mit folgenden Verfahrensschritten:

a) zwei Prüfstreifen des Bahnmaterials werden mit einem ihrer Enden parallel zur Zugrichtung befestigt und mit ihrem losen Ende in entgegengesetzter Richtung durch einen quer zur Zugrichtung angeordneten, aus einem Walzenpaar gebildeten Walzenspalt geführt;

b) die im Walzenspalt übereinanderliegenden Prüfstreifen werden mit einer durch das Walzenpaar vorbestimmten Auflagekraft belastet;

c) gleichzeitig werden die Prüfstreifen parallel zur Zugrichtung mit konstanter Geschwindigkeit bewegt;

d) mittels Kraftaufnehmer wird die Zugkraft gemessen, während die aufeinanderliegenden Prüfstreifen durch den Walzenspalt in entgegengesetzter Richtung gleiten.

2. Meßverfahren zur Bestimmung des Gleitreibungskoeffizienten von einem biegsamen, flächigen Bahnmaterial und einem walzenförmigen Prüfkörper, bei denen die zu messenden gegeneinander reibenden Oberflächen unter einer vorbestimmten Andruckkraft im gleitenden Kontakt stehen, mit folgenden Verfahrensschritten:

a) ein Prüfstreifen des Bahnmaterials wird mit einem seiner Enden parallel zur Zugrichtung befestigt und mit seinem losen Ende durch einen quer zur Zugrichtung angeordneten, aus einem Walzenpaar gebildeten Walzenspalt geführt, wobei eine der Walzen zur Aufnahme des walzenförmigen Prüfkörpers dient;

b) der im Walzenspalt liegende Prüfstreifen wird mit einer durch das Walzenpaar vorbestimmten Auflagekraft belastet;

c) gleichzeitig wird der Prüfstreifen parallel zur Zugrichtung mit konstanter Geschwindigkeit bewegt, wobei die mit dem Prüfkörper versehene Walze mittels drehfester Lagerung gehalten oder entgegen der Bewegungsrichtung des Prüfstreifens mit konstanter Winkelgeschwindigkeit gedreht wird, so daß zwischen Prüfstreifenoberfläche und Prüfkörperoberfläche eine Relativbewegung stattfindet;

d) mittels Kraftaufnehmer wird die Zugkraft gemessen, während sich der Prüfstreifen durch den Walzenspalt bewegt und auf der mit dem walzenförmigen Prüfkörper versehenen Walze gleitet.

3. Meßvorrichtung zur Bestimmung des Gleitreibungskoeffizienten von biegsamen flächigen Bahnmaterialien oder von solchem gegen einen walzenförmigen Prüfkörper, bei denen die zu messenden gegeneinander reibenden Oberflächen unter einer vorbestimmten Andruckkraft im gleitenden Kontakt stehen, bestehend aus:

a) einer Halterung (6),

aa) auf der eine Zentralwalze (9) und eine von der Zentralwalze (9) abhebbare Auflagewalze (8) gelagert sind, die zusammen einen horizontalen zur Zugrichtung senkrecht angeordneten Walzenspalt (14) bilden, daß

ab) auf der Halterung (6) eine Umlenkwalze (10) gelagert ist, die zum Umführen des oberen Prüfstreifens B aus der Horizontalen in die Vertikale dient und daß

ac) die Halterung (6) mit einem Verbindungsteil (16) versehen ist, mit dem die Halterung (6) in Zugrichtung aufhängbar ist;

b) einer Zugvorrichtung (2), die zum vertikalen Verfahren der Halterung (6) dient;

c) einem Kraftaufnehmer (5), der zwischen Verbindungsteil (16) und Zugvorrichtung (2) angeordnet ist;

d) einem Ständer (1) zur Aufnahme der Zugvorrichtung (2);

e) sowie zwei entgegen der Zugrichtung auf dem Ständer (1) angebrachten Befestigungsmittel (3;4) zum Einspannen von Prüfstreifen A und B.

4. Meßvorrichtung nach Anspruch 3 dadurch gekennzeichnet, daß die Auflagewalze (8) auf einem Hebel (7) angeordnet und drehbar gelagert ist und daß der Hebel (7) im Drehpunkt (15) mit der Halterung (6) verbunden ist.

5. Meßvorrichtung nach einem der Ansprüche 3 und 4 dadurch gekennzeichnet, daß der Hebel (7) einen Dorn (11) aufweist, der zur Aufnahme von Zusatzgewichten dient.

6. Meßvorrichtung nach einem der Ansprüche 3 bis 5, dadurch gekennzeichnet, daß der durch Zentralwalze (9) und Auflagewalze (8) gebildete Walzenspalt (14) breiter als das zu prüfende Bahnmaterial ist, und daß die Zugvorrichtung (2) eine Meßstrecke zum Verfahren der Halterung (6) von mehr als 150 mm aufweist.

7. Meßvorrichtung zur Bestimmung des Gleitreibungskoeffizienten von biegsamen, flächigen Bahnmaterialien oder von solchem gegen einen walzenförmigen Prüfkörper, bei denen die zu messenden gegeneinander reibenden Oberflächen unter einer vorbestimmten Andruckkraft im gleitenden Kontakt stehen, bestehend aus:

a) einem Walzenständer (33),

aa) auf dem eine Zentralwalze (9) und eine von der Zentralwalze (9) abhebbare Auflagewalze (8) gelagert sind, die zusammen einen quer zur Zugrichtung angeordneten Walzenspalt (14) bilden und daß

ab) der Walzenständer Mittel, wie Leitblech (31) zum Führen der Prüfstreifen aufweist;

b) einem Führungsbett (27) auf dem der Walzenständer (33) mittig und unverrückbar angebracht ist;

c) zwei auf dem Führungsbett (27) gelagerte, linear verfahrbare Schlitten (28; 29), wobei gleichzeitig beide Schlitten (28; 29) vom Walzenständer (33) aus mit konstanter Geschwindigkeit nach außen verfahrbar sind;

d) Befestigungsmittel (3;4) zum Einspannen von Prüfstreifen A und B, die so auf den Schlitten (28;29) angebracht sind, daß sie mit dem Walzenspalt (14) fluchten;

e) sowie einem Kraftaufnehmer (5), der zwischen Befestigungsmitte (3 oder 4) und Schlitten (28 oder 29) angeordnet ist.

8. Meßvorrichtung nach einem der Ansprüche 3 bis 6 oder 7, dadurch gekennzeichnet, daß die Auflagewalze (8) und/oder die Zentralwalze (9) gummiartige Walzenmäntel aufweisen.

9. Meßvorrichtung nach einem der Ansprüche 7 und 8, dadurch gekennzeichnet, daß der durch Zentralwalze (9) und Auflagewalze (8) gebildete Walzenspalt (14) breiter als das zu prüfende Bahnmaterial ist, und daß die mittels Schlitten (28; 29) verfahrbare Meßstrecke länger als 150 mm ist.

10. Meßvorrichtung nach einem der Ansprüche 3 bis 6 oder 7 bis 9, dadurch gekennzeichnet, daß die Zentralwalze (9) auf ihrem Schaft einen mittels einer Paßfeder (25) befestigten walzenförmigen Prüfkörper (26) aufweist und daß die Zentralwalze (9) drehfest gelagert ist oder durch einen Zentrumsantrieb (24) in Drehung versetzbar ist.

Fig.1

*Fig. 2*

*Fig. 3*

*Fig. 4*

Fig. 5

EP 0 491 234 A2